(19) Europäisches Patentamt
European Patent Office
Office européen des brevets

(11) Publication number: **0 551 662 A1**

(12) # EUROPEAN PATENT APPLICATION

(21) Application number: **92122178.4**

(22) Date of filing: **31.12.92**

(51) Int. Cl.5: **C07D 307/80**, C07D 333/56, A61K 31/34, A61K 31/38

(30) Priority: **13.01.92 GB 9200623**

(43) Date of publication of application: **21.07.93 Bulletin 93/29**

(84) Designated Contracting States: **AT BE CH DE DK ES FR GB GR IE IT LI LU MC NL PT SE**

(71) Applicant: **BAYER AG**

**W-5090 Leverkusen 1 Bayerwerk(DE)**

(72) Inventor: **Butler, Ransohoff, John-E., Dr.**
**Pahlkestrasse 5**
**W-5600 Wuppertal 1(DE)**
Inventor: **Sturton, Graham, Dr.**
**184 Windson Road**
**Maidenhead, Berlshire SL6 2DW(GB)**

(54) **Benzofuranyl- and thiophenylmethylthio-alcanecarboxylic acid derivatives.**

(57) Benzofuranyl- and thiophenylmethylthio-alkanecarboxylic acid derivatives are prepared by brominating appropriate methyl compounds and reacting the methyl bromides thus obtained with mercapto-alkanecarboxylic acid derivatives. The new substances are suitable as NADPH oxidase inhibitors for use in medicaments, in particular for the treatment of acute and chronic inflammatory processes.

EP 0 551 662 A1

The invention relates to benzofuranyl- and thiophenyl-methylthio-alkanecarboxylicacid derivatives, processes for their preparation and their use in medicaments.

Benzofuran and benzothiophene derivatives having lipoxygenase-inhibiting action are described in the publication EP 146,243. The compounds of the general formula (I) according to the invention are partially embraced by the broadest scope of meaning of this application without an actual representative substance being mentioned there. It is additionally known that the NADPH oxidase of phagocytes is the physiological source of the superoxide radical anion ($O_2^-$) and reactive oxygen species derived therefrom which are important in defence against pathogens. Uncontrolled formation leads to tissue damage in inflammatory processes. Many inhibitors of NADPH oxidase which, however, are not selective are known from the literature [cf. Free Radicals in Biology and Medicine, Vol. 8, pp 71-93, 1990].

The invention relates to benzofuranyl- and thenyl-thioalkanecarboxylic acid derivatives of the general formula (I)

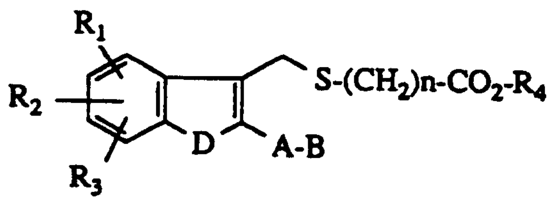
(I)

in which

| | |
|---|---|
| $R^1$, $R^2$ and $R^3$ | are identical or different and represent hydrogen, hydroxyl, straight-chain or branched alkoxy having up to 8 carbon atoms or benzyloxy, |
| n | represents a number 1, 2, 3, 4, 5 or 6, |
| $R^4$ | represents hydrogen, straight-chain or branched alkyl having up to 8 carbon atoms or phenyl, |
| A | represents a direct bond or the -CO- or $-CH_2-$ radical, |
| B | represents aryl having 6 to 10 carbon atoms or a 5- to 7-membered, saturated or unsaturated heterocycle having up to 3 heteroatoms from the series comprising N, S and O, which are optionally monosubstituted to trisubstituted by identical or different substituents from the series comprising carboxyl, halogen, cyano, phenyl, tetrazolyl, thiazolyl, and straight-chain or branched alkyl, alkoxy or alkoxycarbonyl each having up to 8 carbon atoms, or by a group of the formula $-CO-NR^5-SO_2-R^6$, in which |
| $R^5$ | denotes hydrogen or straight-chain or branched alkyl having up to 4 carbon atoms and |
| $R^6$ | denotes straight-chain or branched perfluoroalkyl having up to 6 carbon atoms, or denotes straight-chain or branched alkyl or alkenyl each having up to 6 carbon atoms, and each of which is optionally substituted by phenyl, where the phenyl radical can in turn be monosubstituted to trisubstituted by identical or different substituents from the series comprising halogen, carboxyl, cyano and nitro or by straight-chain or branched alkyl having up to 6 carbon atoms, denotes cycloalkyl having 3 to 7 carbon atoms, or denotes aryl having 6 to 10 carbon atoms or a 5-to 7-membered saturated or unsaturated heterocycle having up to 3 heteroatoms from the series comprising N, S and O, which are optionally monosubstituted to trisubstituted by identical or different substituents from the series comprising halogen, carboxyl, cyano and nitro or by straight-chain or branched alkyl having up to 6 carbon atoms, |
| D | represents an oxygen or sulphur atom |

and their salts.

The benzofuranyl- and thiophenylmethylthio-alkanecarboxylic acid derivatives according to the invention can also be present in the form of their salts. In general, salts with organic or inorganic bases or acids may be mentioned here.

Physiologically acceptable salts are preferred in the context of the present invention. Physiologically acceptable salts of the benzofuranyl- and thiophenyl-methylthio-alkanecarboxylic acid derivatives can be metal or ammonium salts of the substances according to the invention, which contain a free carboxyl group. Those which are particularly preferred are, for example, sodium, potassium, magnesium or calcium salts,

and also ammonium salts which are derived from ammonia, or organic amines, such as, for example, ethylamine, di- or triethylamine, di- or triethanolamine, dicyclohexylamine, dimethylaminoethanol, arginine, lysine or ethylenediamine.

Physiologically acceptable salts can also be salts of the compounds according to the invention with inorganic or organic acids. Preferred salts here are those with inorganic acids such as, for example, hydrochloric acid, hydrobromic acid, phosphoric acid or sulphuric acid, or salts with organic carboxylic or sulphonic acids such as, for example, acetic acid, maleic acid, fumaric acid, malic acid, citric acid, tartaric acid, lactic acid, benzoic acid, or methanesulphonic acid, ethanesulphonic acid, benzenesulphonic acid, toluenesulphonic acid or naphthalenedisulphonic acid.

The compounds according to the invention can exist in stereoisomeric forms which either behave as image and mirror image (enantiomers), or which do not behave as image and mirror image (diastereomers). The invention relates both to the antipodes and to the racemate forms as well as the diastereomer mixtures. The racemate forms, like the diastereomers, can be separated into the stereo-isomerically uniform constituents in a known manner [cf. E.L. Eliel, Stereochemistry of Carbon Compounds, McGraw Hill, 1962].

Heterocycle in general represents a 5- to 7-membered, preferably 5- to 6-membered, saturated or unsaturated ring which as heteroatoms can contain up to 2 oxygen, sulphur and/or nitrogen atoms. 5- and 6-membered rings containing an oxygen, sulphur and/or up to 2 nitrogen atoms are preferred. The following are mentioned as preferred: thienyl, furyl, pyrrolyl, pyrazolyl, pyridyl, pyrimidyl, pyrazinyl, pyridazinyl, thiazolyl, oxazolyl, imidazolyl, pyrrolidinyl, piperidinyl, piperazinyl or tetrazolyl.

Preferred compounds of the general formula (I) are those
in which

$R^1$, $R^2$ and $R^3$ are identical or different and represent hydrogen, hydroxyl, straight-chain or branched alkoxy having up to 6 carbon atoms or benzyloxy,

n represents a number 1, 2, 3, 4 or 5,

$R^4$ represents hydrogen, straight-chain or branched alkyl having up to 6 carbon atoms or phenyl,

A represents a direct bond or the -CO- or $-CH_2-$ radical,

B represents phenyl, pyridyl, thienyl or furyl, which are optionally monosubstituted to trisubstituted by identical or different substituents from the series comprising carboxyl, fluorine, chlorine, bromine, cyano, phenyl, tetrazolyl and thiazolyl, by straight-chain or branched alkyl, alkoxy or alkoxycarbonyl each having up to 6 carbon atoms or by a group of the formula $-CO-NR^5-SO_2R^6$,
in which

$R^3$ denotes hydrogen, methyl or ethyl
and

$R^6$ denotes straight-chain or branched alkyl or perfluoroalkyl each having up to 4 carbon atoms, or
denotes cyclopropyl, cyclopentyl or cyclohexyl, denotes benzyl, phenyl or pyridyl, which are optionally monosubstituted or disubstituted by identical or different substituents from the series comprising fluorine, chlorine, bromine, carboxyl, cyano and nitro or by straight-chain or branched alkyl having up to 4 carbon atoms,

D represents an oxygen or sulphur atom

and their salts.

Particularly preferred compounds of the general formula (I) are those
in which

$R^1$, $R^2$ and $R^3$ are identical or different and
represent hydrogen, hydroxyl or straight-chain or branched alkoxy having up to 4 carbon atoms or benzyloxy,

n represents a number 1, 2, 3 or 4,

$R^4$ represents hydrogen or straight-chain or branched alkyl having up to 4 carbon atoms,

A represents a direct bond or the -CO- or $-CH_2-$ radical,

B represents phenyl or pyridyl, which are optionally monosubstituted to trisubstituted by identical or different substituents from the series comprising carboxyl, cyano, chlorine, bromine and straight-chain or branched alkyl, alkoxy or alkoxycarbonyl having up to 4 carbon atoms or by a group of the formula $-CO-NR^5-SO_2R^6$,
in which

$R^5$ denotes hydrogen or methyl
and

$R^6$ denotes methyl, ethyl, propyl, trifluoromethyl, cyclopropyl or cyclohexyl, or denotes benzyl or phenyl, each of which is optionally monosubstituted or disubstituted by identical or different substituents from the series comprising fluorine, chlorine, bromine, carboxyl, methyl and ethyl,

D represents an oxygen or sulphur atom

and their salts.

Processes for the preparation of the compounds of the general formula (I) have additionally been found, characterised in that compounds of the general formula (II)

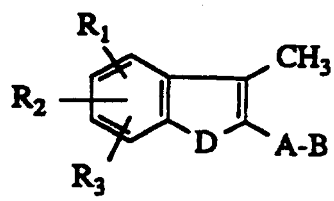

(II)

in which

$R^1$, $R^2$, $R^3$, A, B and D have the abovementioned meaning,

in the case in which $R^1$, $R^2$ and/or $R^3 \neq$ hydroxyl, are converted by reaction with N-bromosuccinimide and in the presence of a catalyst,

in the case in which $R^1$, $R^2$ and/or $R^3$ represents the hydroxyl function, these compounds are first blocked with typical hydroxyl protective groups and then converted with boron tribromide or hydrobromic acid in glacial acetic acid

in inert solvents

to the compounds of the general formula (III)

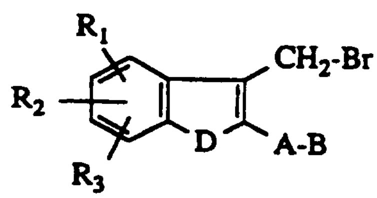

(III)

in which

$R^1$, $R^2$, $R^3$, A, B and D have the abovementioned meaning,

and in a next step the latter are reacted with compounds of the general formula (IV)

$HS-(CH_2)_n-CO_2R^7$ (IV)

in which

n has the abovementioned meaning

and

$R^7$ has the abovementioned meaning of $R^4$, but does not represent hydrogen,

in inert solvents, if appropriate in the presence of a base, under a protective gas atmosphere,

and in the case of the acids the esters are hydrolysed,

and in the case of the free hydroxyl functions ($R^1$, $R^2$ and/or $R^3$ = OH) the protective groups are removed by a customary method,

and in the case in which B represents one of the above-mentioned cyclic radicals which are substituted by the group of the formula $-NR^5-SO_2R^6$, using sulphonamides of the formula (V)

$HNR^5-SO_2-R^6$ (V)

in which

$R^5$ and $R^6$ have the abovementioned meaning,

starting from the free carboxylic acid, if appropriate in the presence of a base and/or of an auxiliary, an

amidation follows.

The process according to the invention can be illustrated by way of example by the following equation:

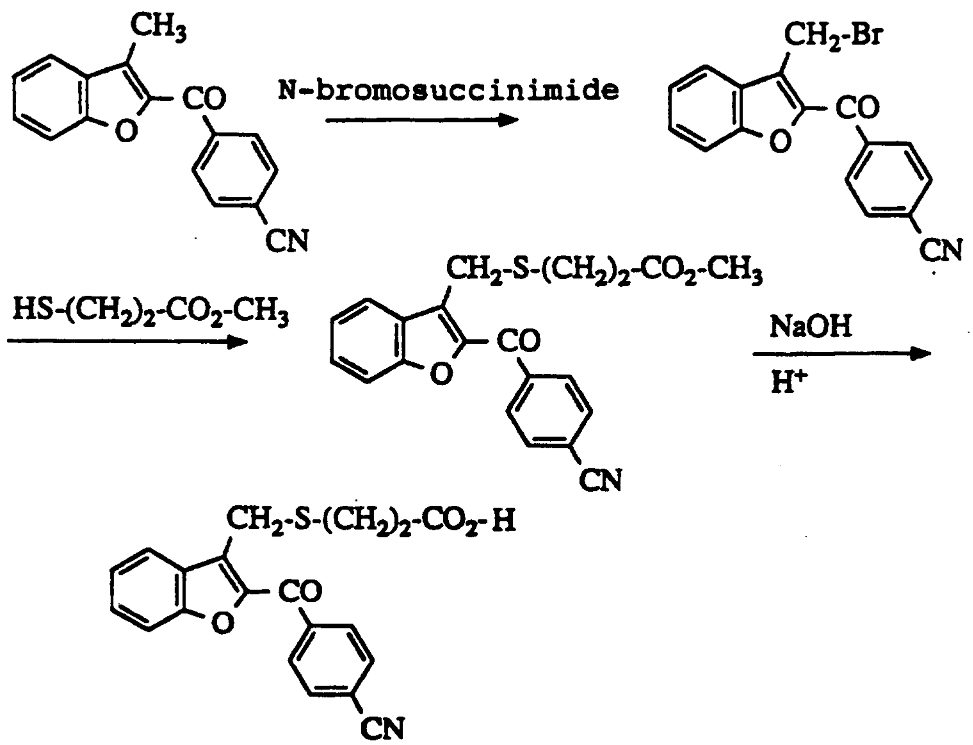


Hydroxyl protective group in the context of the above-mentioned definition in general represents a protective group from the series comprising: trimethylsilyl, tert-butyl-dimethylsilyl, benzyl, 4-nitrobenzyl, 4-methoxybenzyl, acetyl, tetrahydropyranyl and benzoyl.

Suitable solvents for the thioetherification are generally customary organic solvents which do not change under the reaction conditions. These preferably include ethers such as diethyl ether, dioxane, tetrahydrofuran or glycol dimethyl ether, acetone, dimethylformamide or methyl isobutyl ketone.
Suitable solvents for the bromination are halogenohydrocarbons such as dichloromethane, trichloromethane, tetrachloromethane, dichloroethylene, trichloroethylene or chlorobenzene. Carbon tetrachloride is preferred for the bromination with N-bromosuccinimide, dichloromethane for the bromination with boron tribromide and glacial acetic acid for the bromination with hydrobromic acid. Acetone or methyl isobutyl ketone are preferred for thioetherification.

Suitable catalysts for bromination are generally radical generators such as, for example, dibenzoyl peroxide or azobisisobutyronitrile. Dibenzoyl peroxide is preferred.

The catalyst is employed in an amount from 0.001 mol to 0.2 mol, preferably from 0.1 mol to 0.05 mol, relative to 1 mol of the compounds of the general formula (II).

Suitable bases for thioetherification are generally inorganic or organic bases. These preferably include alkali metal hydroxides such as, for example, sodium hydroxide or potassium hydroxide, alkaline earth metal hydroxides such as, for example, barium hydroxide, alkali metal carbonates such as sodium carbonate, potassium carbonate, alkaline earth metal carbonates such as calcium carbonate, or alkali metal or alkaline earth metal alkoxides such as sodium methoxide or potassium methoxide, sodium ethoxide or potassium ethoxide or potassium tert-butoxide, or organic amines (trialkyl($C_1$-$C_6$)amines) such as triethylamine, or heterocycles such as 1,4-diazabicyclo[2.2.2]octane (DABCO), 1,8-diazabicyclo[5.4.0]undec-7-ene (DBU), pyridine or methylpiperidine. It is also possible to employ alkali metals, such as sodium or its hydrides such as sodium hydride, as bases. Potassium carbonate is preferred.

The base is employed in an amount from 1 mol to 10 mol, preferably from 2.0 mol to 2.1 mol, relative to 1 mol of the compounds of the general formula (III).

Bromination is generally carried out in a temperature range from -30 °C to +150 °C, preferably from -20 °C to +50 °C.

Bromination is generally carried out at normal pressure. However, it is also possible to carry out bromination at elevated pressure or at reduced pressure (for example in a range from 0.5 to 5 bar).

Thioetherification is in general carried out in a temperature range from +10 °C to +150 °C, preferably from +20 °C to +100 °C.

Thioetherification is generally carried out at normal pressure. However, it is also possible to carry out thioetherification at elevated pressure or at reduced pressure (for example in a range from 0.5 to 5 bar).

In general, 0.5 to 5, preferably 1 to 2, mol of halide (III) is employed, relative to 1 mol of the reaction component. The base is in general employed in an amount from 0.5 to 5 mol, preferably from 1 to 3 mol relative to the halide.

The removal of the protective groups is carried out by a customary method, for example by hydrogenolytic cleavage of the benzyl ethers in the abovementioned inert solvents in the presence of a catalyst using hydrogen gas [cf. additionally Th. Green: "Protective Groups in Organic Synthesis", J. Wiley & Sons, 1981, New York].

The tert-butyl group is preferably removed with trifluoroacetic acid in dichloromethane, and the methyl ester groups in tetrahydrofuran with a methanolic sodium hydroxide solution.

The compounds of the general formula (II) are known in some cases [cf. EP 399,773; EP 146,243 and J. Med. Chem. 1978, Vol. 21, No. 4, pp. 348-352] and in the case in which A represents the -CO- group, for example, can be prepared

by reacting compounds of the general formula (VI)

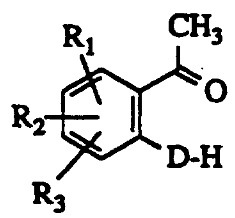

(VI)

in which

$R^1$, $R^2$, $R^3$ and D have the abovementioned meaning,

with compounds of the general formula (VII)

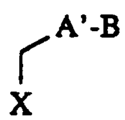

(VII)

in which

A' represents the -CO- group,

B has the abovementioned meaning

and

X represents a typical leaving group such as, for example, chlorine, bromine, iodine, tosylate or mesylate, preferably bromine,

in inert solvents, if appropriate in the presence of a base

and in the case in which A represents a bond

compounds of the general formula (VI) are converted by reaction with compounds of the general formula (VIII)

$B\text{-}CH_2\text{-}Y$ (VIII)

in which

B has the abovementioned meaning

and

Y has the abovementioned meaning of X, but preferably represents chlorine,

in inert solvents, in the presence of a base, to the compounds of the general formula (IX)

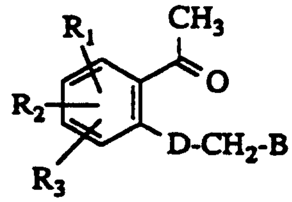

(IX)

in which

$R^1$, $R^2$, $R^3$, B and D have the abovementioned meaning,

and then carrying out a cyclisation by a customary method, preferably using acetic anhydride and in the case in which A represents the $-CH_2-$ group,

first reacting the compounds of the general formula (VI) and (VII) as described above and then reducing the keto function (A' = -CO) according to customary methods.

Suitable solvents for the reaction with the compounds of the general formulae (VI), (VII), (VIII) and (IX) are the abovementioned solvents and bases, preferably acetone and potassium carbonate.

The base is employed in an amount from 1 mol to 10 mol, preferably from 1.0 mol to 2.1 mol, relative to 1 mol of the compounds of the general formula (VI).

The reactions in general proceed in a temperature range from +30°C to +100°C, preferably from +40°C to +80°C and at normal pressure.

The cyclisation in general proceeds in a temperature range from +30°C to +180°C, preferably from +60°C to +120°C and at normal pressure.

The reduction of the keto function is in general carried out using the customary reducing agents, for example with hydrides such as sodium cyanoborohydride or sodium borohydride, preferably with sodium cyanoborohydride, in inert solvents such as ethers, hydrocarbons or alcohols or their mixtures, preferably in ethers such as, for example, diethyl ether, tetrahydrofuran or dioxane, or alcohols such as ethanol, in a temperature range from 0°C to +150°C, preferably from +20°C to +50°C.

The process according to the invention is in general carried out at normal pressure. However, it is also possible to carry out the process at elevated pressure or at reduced pressure (for example in a range from 0.5 to 5 bar).

The compounds of the general formulae (V), (VI), (VII), (VIII) and (IX) are known per se or can be prepared by a customary method [cf. Beilstein 7, 271; Beilstein 8, 85; Helv. Chim. Acta 50, (1967) 628, 637, 2233; 52 (1969), 887, 888; J. Org. Chem. 5 (1940), 54, 56; EP 165,810; Houben-Weyl, Vol. IX p. 407 ff.].

The compounds of the general formula (III) are known in some cases [cf., for example, US 4,137,414, US 413,424; J. Med. Chem. 1978, Vol. 21 No. 4] or are new and can then be prepared, for example, by the abovementioned process.

The compounds of the general formula (IV) are known [cf. Beilstein 3, 255, MSD Book 2,2447 B].

Suitable bases for the hydrolysis are the customary inorganic bases. These preferably include alkali metal hydroxides or alkaline earth metal hydroxides such as, for example, sodium hydroxide, potassium hydroxide or barium hydroxide, or alkali metal carbonates such as sodium carbonate or potassium carbonate or sodium hydrogen carbonate, or alkali metal alkoxides such as sodium methoxide, sodium ethoxide, potassium methoxide, potassium ethoxide or potassium tert-butoxide. Sodium hydroxide or potassium hydroxide are particularly preferably employed.

Suitable solvents for the hydrolysis are water or the organic solvents customary for hydrolysis. These preferably include alcohols such as methanol, ethanol, propanol, isopropanol or butanol, or ethers such as tetrahydrofuran or dioxane, or dimethylformamide, or dimethyl sulphoxide. Alcohols such as methanol, ethanol, propanol or isopropanol are particularly preferably used. It is also possible to employ mixtures of the solvents mentioned.

The hydrolysis can also be carried out with acids such as, for example, trifluoroacetic acid, acetic acid, hydrochloric acid, hydrobromic acid, methanesulphonic acid, sulphuric acid or perchloric acid, preferably with trifluoroacetic acid.

The hydrolysis is in general carried out in a temperature range from 0°C to +100°C, preferably from +20°C to +80°C.

In general, the hydrolysis is carried out at normal pressure. However, it is also possible to work at reduced pressure or at elevated pressure (for example from 0.5 to 5 bar).

When carrying out the hydrolysis, the base is in general employed in an amount from 1 to 3 mol, preferably from 1 to 1.5 mol, relative to 1 mol of the ester. Molar amounts of the reactants are particularly preferably used.

When carrying out the preparation of the compounds of the formula (I), in the first step the carboxylates of the compounds according to the invention are formed as intermediates which can be isolated. The acids according to the invention are obtained by treating the carboxylates with customary inorganic acids. These preferably include mineral acids such as, for example, hydrochloric acid, hydrobromic acid, sulphuric acid or phosphoric acid. In this connection, it has proved advantageous in the preparation of the carboxylic acids to acidify the basic reaction mixture from the hydrolysis in one step without isolation of the carboxylates. The acids can then be isolated in a customary manner. In the case of basic heterocycles, the salts of the heterocycles with the inorganic acids can also be obtained by treating the solutions of the carboxylates with the abovementioned acids.

The amidation/sulphoamidation is in general carried out in one of the abovementioned solvents, preferably in dichloromethane. It may optionally proceed, starting from the free carboxylic acid, via an activated stage, for example via the corresponding acid halides, which can be prepared from the corresponding acids by reaction with thionyl chloride, phosphorus trichloride, phosphorus pentachloride, phosphorus tribromide or oxalyl chloride. Preferably, the activated stages are prepared from the corresponding acids using dicyclohexylcarbodiimide, N-(3-dimethylaminopropyl)-N'-ethylcarbodiimide hydrochloride or carbonyldiimidazole and reacted in situ with the sulphonamides.

The amidation and the sulphoamidation are in general carried out in a temperature range from -20 °C to +80 °C, preferably from -10 °C to +30 °C and at normal pressure.

In addition to the abovementioned bases, suitable bases for these reactions are preferably triethylamine and/or dimethylaminopyridine, DBU or DABCO.

The base is employed in an amount from 0.5 mol to 10 mol, preferably from 1 mol to 2 mol, relative to 1 mol of the compounds of the general formula (V).

Acid-binding agents which can be employed for the sulphoamidation are alkali metal or alkaline earth metal carbonates such as sodium carbonate, potassium carbonate, alkali metal or alkaline earth metal hydroxides such as, for example, sodium hydroxide or potassium hydroxide, or organic bases such as pyridine, triethylamine, N-methylpiperidine, or bicyclic amidines such as 1,5-diazabicyclo[3.4.0]-non-5-ene (DBN) or 1,5-diazabicyclo[3.4.0]undec-5-ene (DBU). Potassium carbonate is preferred.

Suitable dehydrating reagents are carbodiimides such as, for example, diisopropylcarbodiimide, dicyclohexylcarbodiimide or N-(3-dimethylaminopropyl)-N'-ethylcarbodiimide hydrochloride or carbonyl compounds such as carbonyldiimidazole or 1,2-oxazolium compounds such as 2-ethyl-5-phenyl-1,2-oxazolium-3-sulphonate or propanephosphonic anhydride or isobutyl chloroformate or benzotriazolyloxytris-(dimethylamino)phosphonium hexafluorophosphate or diphenyl phosphoramidate or methanesulphonyl chloride, if appropriate in the presence of bases such as triethylamine or N-ethylmorpholine or N-methylpiperidine or dicyclohexylcarbodiimide and N-hydroxysuccinimide [cf. J.C. Sheehan, S.L. Ledis, J. Am. Chem. Soc. 95, 875 (1973); F.E. Frerman et al., J. Biol. Chem. 225, 507 (1982) and N.B. Benoton, K. Kluroda, Int. Pept. Prot. Res. 13, 403 (1979), 17, 187 (1981)].

The acid-binding agents and dehydrating reagents are in general employed in an amount from 0.5 to 3 mol, preferably from 1 to 1.5 mol, relative to 1 mol of the corresponding carboxylic acids.

The benzofuranyl- and thenylthio-alkanecarboxylic acid derivatives according to the invention exhibit an unforeseeable, useful pharmacological spectrum of action.

The compounds according to the invention specifically inhibit the NADPH oxidase of phagocytes, without impairing other cellular functions, such as, for example, degranulation or respiratory chain phosphorylation.

They can therefore be employed in medicaments for controlling acute and chronic inflammatory processes.

The compounds according to the invention are preferably suitable for the treatment and prevention of acute and chronic inflammations of the airways, such as emphysema, alveolytis, shock lung, asthma, bronchitis, of the joints, such as rheumatism and of the vessels, such as arteriosclerosis, arthrosis, inflammations of the gastrointestinal tract and myocarditis. The compounds according to the invention are additionally suitable for reducing the damage to infarct tissue after reoxygenation. In this case, the simultaneous administration of allopurinol to inhibit xanthine oxidase is of advantage. Combination therapy with superoxide dismutase is also of use. The compounds according to the invention are novel oxygen radical scavengers.

## Test description

1. Preparation of human PMN

Blood was taken from healthy subjects by venous puncture and neutrophils were purified by dextran

sedimentation and resuspended in the buffered medium.

2. Inhibition of FMLP-stimulated production of superoxide radical anions.

Neutrophils ($2.5 \times 10^5$ $ml^{-1}$) were mixed with the compounds according to the invention in dimethyl sulphoxide (DMSO), the final concentration of the compounds according to the invention being 10 $\mu$M and the DMSO concentration being 1% v/v. After addition of luminol, final concentration about $10^{-5}$ M and 33 $\mu$g/ml of cytochalasin B, the cells were stimulated with a final concentration of about $5 \times 10^{-8}$ M FMLP. Measurement of the chemiluminescence peaks was carried out using an LKB 1251 luminometer. The inhibition of the superoxide and the reduction of cytochrome C by FMLP-stimulated cells was likewise measured.

Neutrophils ($2.6 \times 10^5$ $ml^{-1}$) were mixed with the compounds according to the invention as in the chemiluminescence experiment. Cytochrome C with a concentration of $3.8 \times 10^{-8}$ M FMLP is also present. The superoxide-dependent cytochrome C reduction was measured using a Molecular Devices Thermomax plate reader.

The inhibition of superoxide production was calculated as follows:

$$\left[ 1 - \left( \frac{Ex}{E_o} \right) \times 100 \right]$$

Ex = Emission of the cuvette containing the compound according to the invention

$E_0$ = Emission of the control cuvette

FMLP-induced chemiluminescence of polymorphonuclear granulocytes, % inhibition on administration of 10 $\mu$M of the test substance and $IC_{50}$ [$\mu$M]

Table A

| Example No. | % Inhibition | $IC_{50}$ |
|---|---|---|
| 17 | 96 ± 2 | 0.7 |
| 18 | 77 ± 8 | |
| 19 | 43 ± 15 | |
| 20 | 59 ± 18 | |
| 21 | 51 ± 7 | |
| 22 | | 4.0 |
| 23 | | 2.5 |
| 24 | | 5.8 |

3. Inhibition of myeloperoxidase

The compounds according to the invention were tested for their action on human neutrophil myeloperoxidase activity by the established method of Bos, A.J. et al., Infection and Immunity, 1981, 32 (2), 427-431.

4. Xanthine oxidase assay

Equal parts of the substrate (7.5 mM hypoxanthine) were incubated at 25 °C with the compounds according to the invention or carrier, and $10^{-5}$ M luminol and the enzyme xanthine oxidase were added immediately before starting the luminescence measurement. The formation of superoxide was then measured for 140 sec in the 1251 luminometer. The activity was in each case measured against xanthine oxidase in the presence or absence of the compounds according to the invention by monitoring the growth of the absorption at 292 nm due to the formation of uric acid.

5. Determination of the specificity

When neutrophils are activated by PMA or FMLP, they release the lysosomal enzyme beta-glucuronidase. Preliminary experiments showed that in the presence of cytochalasin B, FMLP exhibit a stronger stimulating effect than PMA. Using a concentration of $10^6$ cells $ml^{-1}$, the compounds according to the invention were tested in parallel using a cell preparation thereof to gauge their capability to inhibit FMLP-induced superoxide production and FMLP-induced beta-glucuronidase release.

6. NADPH oxidase assay

Neutrophils were stimulated with PMA and treated with ultrasound, and a membrane fraction was prepared. The oxidation of exogenous NADPH by this fraction was observed spectrophotometrically by decrease in the absorption at 340 nm. The compounds were measured at a final concentration of 33 $\mu$M.

% inhibition of NADPH oxidase in membrane fractions, measured at a final concentration of 33 $\mu$M of the test substance

Table B

| Ex. No. | % inhibition of NADPH oxidase |
|---|---|
| 17 | 21 ± 7 |
| 31 | 20 |

7. Mitochondrial substrate oxidation assay

A mitochondrial fraction was prepared from finely-cut guinea pig hearts. The oxidation of both nicotinamide adenine dinucleotides, attributed to NADH, was observed with the aid of a Clandon Scientific Model 53 oxygen electrode. The compounds according to the invention were tested to gauge their inhibition of cell respiration by measurement of their effect on the oxidation of these two substances at a concentration of up to 100 $\mu g/ml^{-1}$.

% inhibition of NADPH oxidation by mitochondria at a final concentration of 300 $\mu$M of the test substance

Table C

| Ex. No. | % inhibition (mitochondrial respiration) |
|---|---|
| 17 | 24 ± 2 |
| 31 | 0 ± 7 |

The new active compounds can be converted in a known manner into the customary formulations, such as tablets, coated tablets, pills, granules, aerosols, syrups, emulsions, suspensions and solutions, using inert, non-toxic, pharmaceutically suitable excipients or solvents. In this connection, the therapeutically active compound should in each case be present in a concentration of about 0.5 to 90% by weight of the total mixture, i.e. in amounts which are sufficient in order to achieve the dosage range indicated.

The formulations are prepared, for example, by extending the active compounds with solvents and/or excipients, if appropriate using emulsifiers and/or dispersants, where, for example, in the case of the use of water as a diluent, organic solvents can be used as auxiliary solvents if appropriate.

Administration is carried out in a customary manner, preferably orally or parenterally, in particular perlingually or intravenously.

In the case of parenteral administration, solutions of the active compound can be employed using suitable liquid vehicles.

In general, it has proved advantageous on intravenous administration to administer amounts from about 0.001 to 10 mg/kg, preferably about 0.01 to 5 mg/kg of body weight to achieve effective results, and on oral administration the dosage is about 0.01 to 25 mg/kg, preferably 0.1 to 10 mg/kg of body weight.

In spite of this, it may be necessary to depart from the amounts mentioned, in particular depending on the body weight or the type of application route, on individual behaviour towards the medicament, the manner of its formulation and the time or interval at which administration takes place. Thus, in some cases it may be sufficient to manage with less than the abovementioned minimum amount, while in other cases the upper limit mentioned must be exceeded. In the case of administration of relatively large amounts, it is advisable to divide these into several individual doses over the course of the day.

Solvents

A = $CH_2Cl$

Al = $CH_2Cl_2/CH_3OH$ 99:1

A5 = $CH_2Cl_2/CH_3OH$ 95:5

A10 = $CH_2Cl_2/CH_3OH$ 9:1
B = ether/petroleum ether 1:1
No details: RP-HPLC (C18 column) eluent 10-90% acetonitrile in phosphate buffer (0.01 M, pH = 7.5)
To illustrate the substituents' positions in the examples mentioned, the basic structure of the compounds according to the invention is indicated with numbering:

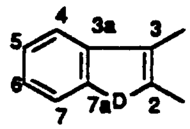

Starting compounds

Example I

2-(4-Bromobenzoyl)-3-methyl-benzo[b]furan

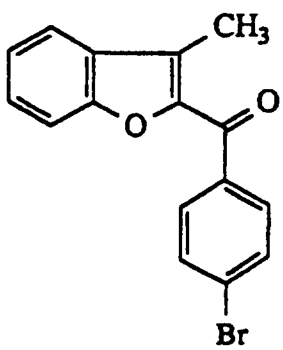

Equivalent amounts, 10.8 g (0.079 mol) of 2-hydroxyacetophenone and 22.1 g (0.079 mol) of ω-bromo-4-bromoacetophenone, are dissolved in 50 ml of acetone and 22.0 g (0.159 mol) of potassium carbonate are added. The suspension is heated under reflux for 16 hours. The mixture is filtered while hot and washed with hot acetone, and the collected extracts are freed from the solvent in vacuo. The crude product is recrystallised in methanol or, if appropriate, purified by chromatography (silica gel 60).
Yield: 17.2 g (69%)
M.p.: 113 °C
$R_f$: 0.84 (A)
The compounds shown in Table I are prepared in analogy to the procedure of Example I:

## Table I:

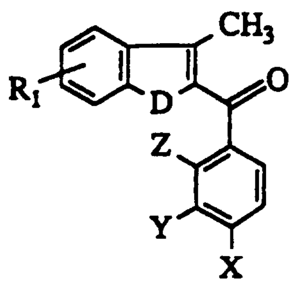

| Ex. No. | D | $R^1$ | Y | X | Z | $R_f$ | M.p. °C | Yield (% of theory) |
|---|---|---|---|---|---|---|---|---|
| II | O | H | H | H | H | 0.83 (A) | | 95 |
| III | O | H | H | CN | H | 0.27 (A) | | 19 |
| IV | O | H | -CN | H | H | 0.26 (A) | | 15 |
| V | O | H | $-CO_2CH_3$ | $OCH_3$ | H | 0.47 (A) | | 79 |
| VI | O | H | H | $CO_2CH_3$ | H | 0.57 (A) | | 47 |
| VII | O | 4-OH | H | $CH_3$ | H | 0.62 (A) | | 29 |
| VIII | O | $6-OCH_2C_6H_5$ | H | $CH_3$ | H | 0.70 (A) | | 28 |
| IX | O | $6-OCH_3$ | $-CH_3$ | H | H | 0.64 (A) | | 55 |
| X | O | $6-OCH_3$ | H | $OCH_3$ | $CH_3$ | 0.56 (A) | | 85 |
| XI | O | $p-OCH_3$ | H | Br | H | 0.71 (A) | | 66 |
| XII | O | H | H | Cl | H | | 107 | 56 |
| XIII | O | $4-O-CH_2C_6H_5$ | H | $CH_3$ | H | 0.82 (A) | | |
| XIV | O | $5-OCH_3$ | H | $CH_3$ | H | 0.67 (B) | | |
| XV | O | $6-OCH_3$ | H | $CH_3$ | H | 0.78 (A) | | 57 |
| XVI | S* | H | H | Cl | H | 0.85 (A) | 101 | 89 |

Retention time in HPLC, $SiO_2$ 60 $(CH_3/CN)/H_2O$ gradient/
*benzo[b]thiophene

Example XVII

2-(4-Bromobenzoyl)-3-bromomethyl-benzo[b]furan

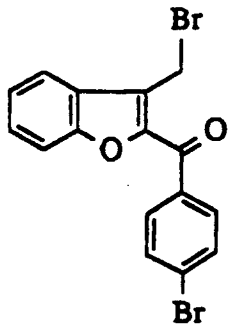

16.9 g (0.054 mol) of the compound from Example I are dissolved in 100 ml of carbon tetrachloride, 9.5 g (0.054 mol) of N-bromosuccinimide are added and the mixture is treated with 0.3 g of dibenzoyl peroxide and heated to reflux for 8 hours. The mixture is filtered while hot, the solvent is distilled off in vacuo and the residue is optionally chromatographed.
$R_f$ = 0.91 ($CH_2Cl_2$, $SiO_2$)
Melting point: 138 °C
Yield: 14.7 g (69% of theory)

Example XVIII

3-Bromomethyl-2-(4-methylbenzoyl)-6-hydroxybenzo[b]furan

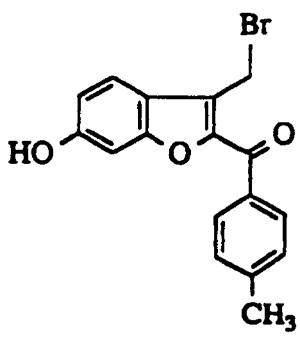

a)
14.6 g (0.041 mol) of the compound of Example XV are dissolved in 50 ml of dichloromethane, cooled to -20 °C and 203 ml (0.203 mol) of $BBr_3$ (1 M solution in $CH_2Cl_2$) and added dropwise. After warming to room temperature overnight, the mixture is poured onto ice and subsequently extracted three times with ethyl acetate. The combined organic phases are dried using $Na_2SO_4$ and freed from solvent in vacuo, and the residue is crystallised from $CH_2Cl_2$.
Yield: 10.5 g (75% of theory)
$R_f$ = 0.16 (A)
b)
The title compound can also be prepared by addition of 20 ml (33%) of HBr in glacial acetic acid to 1.75 g of the compound from Example VIII dissolved in 150 ml of warm glacial acetic acid. The solution is warmed to 60 °C for 2 hours and then concentrated in vacuo. Dilution with cold water gives the solid product, which is then filtered, washed with water and dried over $P_2O_5$ in vacuo.
Yield: quantitative

The compounds shown in Table 2 are prepared in analogy to the procedure of Examples XVII and XVIII:

## Table II:

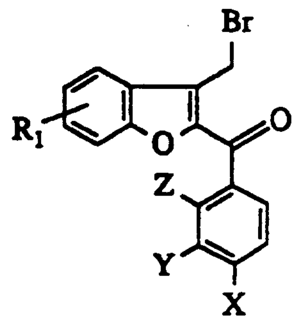

| Ex. No. | $R^1$ | Y | X | Z | $R_f$(*) | M.p.°C | Yield (% of theory) |
|---|---|---|---|---|---|---|---|
| XIX | H | H | H | H | 0.92 (A) | 102 | 37 |
| XX | H | H | CN | H | 0.64 (A) | | 58 |
| XXI | H | CN | H | H | 0.64 (A) | | 40 |
| XXII | H | $CO_2CH_3$ | $OCH_3$ | H | 0.24 (A) | | 57 |
| XXIII | H | H | $CO_2CH_3$ | H | 0.60 (A) | | 38 |
| XXIV | 4-OH | H | $CH_3$ | H | 0.56 (A1) | | 20 |
| XXV | 6-OH | H | $CH_3$ | H | 0.28 (A) | | 41 |
| XXVI | 5-OH | H | $CH_3$ | H | 0.22 (A) | | 52 |
| XXVII | 6-O-$CH_2C_6H_5$ | H | $CH_3$ | H | 0.74 (A) | | 28 |
| XXVIII | 6-OH | $CH_3$ | H | H | 0.32 (A1) | | 66 |
| XXIX | 6-OH | H | $OCH_3$ | $CH_3$ | 0.20 (A) | | 78 |
| XXX | H | H | Cl | H | 0.85 (a) | 128 | 41 |
| XXXI | 4-$OCH_2C_6H_5$ | H | $CH_3$ | H | 0.91 (B) | | 19 |
| XXXII | 6-$OCH_3$ | H | $CH_3$ | H | 0.78 (A) | | 39 |
| XXXIII | 5-$OCH_3$ | H | $CH_3$ | H | 0.86 (A) | | 31 |
| XXXIV | 6-$OCH_3$ | H | Br | H | 0.87 (A) | | 80 |
| XXXV | 6-$OCH_3$ | $H_3C$ | H | H | 0.85 (A1) | | 48 |
| XXXVI | 6-$OCH_3$ | H | H | $CH_3$ | 0.82 (A) | | 67 |

Preparation Examples

Example 1

2-(4-Bromobenzoyl)-3-[1-(2-methoxycarbonylethyl)thiomethyl]benzo[b]furan

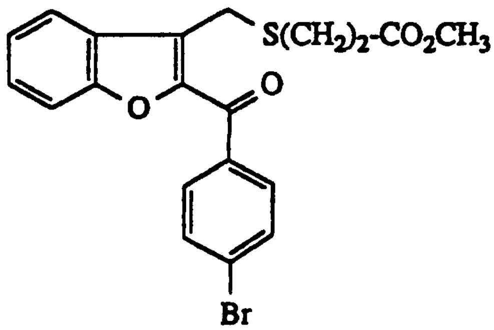

5.0 g (0.013 mol) of the compound from Example XVII, 1.7 g (0.014 mol) of methyl 3-mercaptopropionate and 1.8 g (0.013 mol) of $K_2CO_3$ are suspended in 50 ml of acetone or MIBK, 0.5 g of potassium iodide are added, and the mixture is heated to reflux under argon for eight hours. After cooling, it is filtered, the solid is washed with acetone and the combined extracts are concentrated. The residue is taken up in $CH_2Cl_2$, and the solution is washed several times with bicarbonate solution, dried over $MgSO_4$ and concentrated in a rotary evaporator. The product is purified, if appropriate, by chromatography (silica gel 60).
Yield: quantitative
$R_f$ = 0.51 (A)
The examples shown in Table 1 are prepared in analogy to the procedure of Example 1:

**Table 1:**

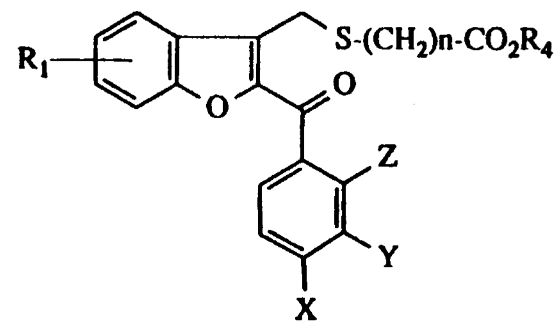

| Ex. No. | $R^1$ | n | Y | X | Z | $R^4$ | Yield (% of theory) | $R_f$ (*) |
|---|---|---|---|---|---|---|---|---|
| 2 | H | 2 | H | H | H | $-CH_3$ | 90 | 0.58 (A) |
| 3 | H | 2 | H | CN | H | $-CH_3$ | 66 | 0.42 (A) |
| 4 | H | 2 | -CN | H | H | $-CH_3$ | 90 | 0.34 (A) |
| 5 | H | 2 | $-CO_2CH_3$ | $-OCH_3$ | H | $-C(CH_3)_3$ | 85 | 0.15 (A) |

Continuation of Table 1:

| Ex. No. | $R^1$ | n | Y | X | Z | $R^4$ | Yield (% of theory) | $R_f$ (*) |
|---|---|---|---|---|---|---|---|---|
| 6 | H | 2 | H | $-CO_2CH_3$ | H | $-CH_3$ | 68 | 0.18 (A) |
| 7 | 4-OH | 2 | H | $CH_3$ | H | $-C(CH_3)_3$ | 76 | 0.27 (A) |
| 8 | 6-OH | 2 | H | $CH_3$ | H | $-C(CH_3)_3$ | 45 | 0.28 (A) |
| 9 | 5-OH | 2 | H | $CH_3$ | H | $-CH_3$ | 34 | 0.58 (A5) |
| 10 | $O-CH_2-C_6H_5$ | 2 | H | $CH_3$ | H | $-CH_3$ | quant. | 0.38 (A) |
| 11 | 6-OH | 2 | $-CH_3$ | H | H | $-CH_3$ | 77 | 0.12 (A) |
| 12 | 6-OH | 2 | H | H | $CH_3$ | $-CH_3$ | 81 | 0.31 (A1) |
| 13 | 6-OH | 1 | H | $CH_3$ | H | $-C_2H_5$ | 49 | 0.09 (A) |

Example 14 and Example 15

2-[3-Carboxy-4-methoxy-benzoyl]-3-[(2-tert.-butoxycarbonylethyl)thiomethyl]benzo[b]furan

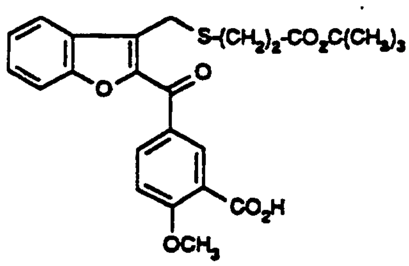

(Example 14)

2-[3-Carboxy-4-methoxy-benzoyl]-3-[(2-carboxyethyl)thiomethyl]benzo[b]furan

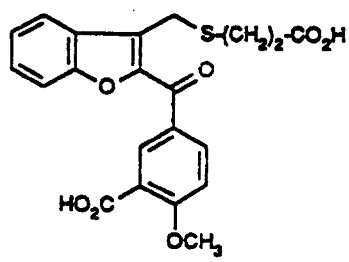

(Example 15)

49.2 g (0.100 mol) of the ester from Example 5 are dissolved in 400 ml of methanol/tetrahydrofuran (5:3) and the mixture is cooled to 0 °C and then treated with 55.9 ml of 2N NaOH. After stirring at room temperature for 24 hours, the mixture is concentrated in vacuo, the residue is dissolved in water, and the solution is separated from insoluble materials on kieselguhr and finally adjusted to pH = 7.0 using phosphate buffer and 0.1N HCl. The aqueous phase is extracted with ethyl acetate several times, and the organic extracts are washed with buffer solution, dried over $MgSO_4$ and concentrated. The residue is recrystallised from isopropyl ether. 26.1 g (55% of theory) of the monoester (Example 14) are isolated with a small amount (4 g, 9.6%) of the dicarboxylic acid (Example 15), which is obtained in pure form from the aqueous mother liquor after acidification (pH ≦ 3).

Example 14: Yield: 58% of theory
$R_f$ = 0.24 ($CH_2Cl_2/CH_3OH$ 99:1)

Example 15: Yield: 10% of theory
$R_f$ = 0.78

Example 16

2-[4-Methoxy-3-(2-tolylsulphonamido-carbonyl)-benzoyl]-3-[(2-tert.- butoxycarbonyl-ethyl)thiomethyl]benzo-[b]furan

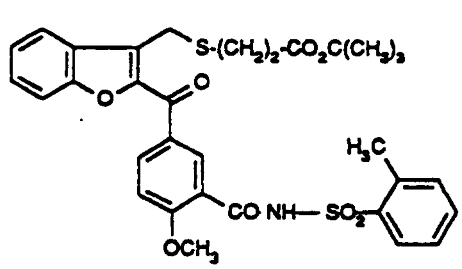

1.8 g (0.011 mol) of o-tolylsulphonamide, 5.0 g (0.011 mol) of carboxylic acid from Example 11, 1.3 g (0.011 mol) of dimethylaminopyridine and 2.4 g (0.013 mol) of N-ethyl-N'-(3-dimethylaminopropyl)-carbodiimide hydrochloride are dissolved in $CH_2Cl_2$. The solution is stirred for 12 h with exclusion of moisture. For working-up, it is shaken in succession twice each time with 1N HCl and water, and the organic phase is dried using $Na_2SO_4$ and concentrated in vacuo. The residue is recrystallised from methanol/$CH_2Cl_2$.
Yield: 99% of theory
$R_f$ = 0.39 ($CH_2Cl_2$/$CH_3OH$ 99:1)

Example 17

2-[4-Methoxy-3-(2-tolylsulphonamide-carbonyl)-benzoyl]-3-[(2-carboxyethyl)thiomethyl]benzo[b]furan

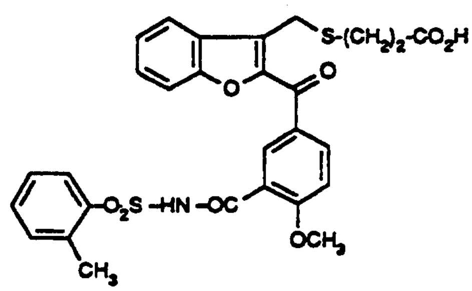

4.8 g (0.008 mol) of t-Butyl ester (Example 16) are dissolved in 50 ml of pure trifluoroacetic acid and the solution is allowed to stand at room temperature for 2 h. The trifluoroacetic acid is distilled off in vacuo and the residue is crystallised from dichloromethane/ethyl acetate. The product is carefully washed with water and dried over $P_2O_5$ in vacuo.

Methyl esters are dissolved in THF analogously to Example 14, treated with 1.1 equivalent (in the case of hydroxybenzo[b]furan 2.2 equivalents are required) of 1N NaOH in methanol and stirred overnight. The solution is concentrated in vacuo and the residue is dissolved in water. The solution is then washed several times with diethyl ether and acidified with HCl (1N). The product is filtered off or, if appropriate, extracted with ethyl acetate, dried with $MgSO_4$ and freed from solvent in vacuo.
Yield: 76% of theory
$R_f$ = 0.46 ($CH_2Cl_2$)
The examples shown in Table 2 are prepared from the carboxylic acid esters described above (Examples 1 - 17) by hydrolysis (see general procedure):

Table 2:

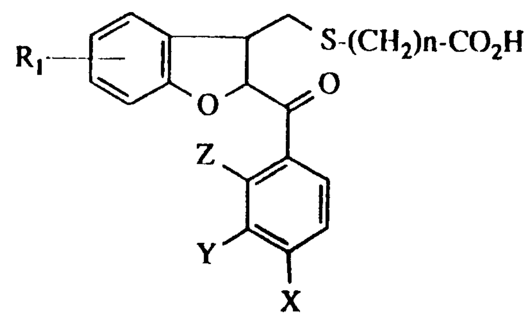

| Ex. No. | $R^1$ | n | Y | X | Z | Yield (% of theory) | $R_f$ (*) |
|---|---|---|---|---|---|---|---|
| 18 | H | 2 | H | -CN | H | 50 | 6.281 |
| 19 | H | 2 | -CN | H | H | 52 | 6.208 |
| 20 | H | 2 | H | H | H | 38 | 0.49 (A5) |

**Continuation of Table 2:**

| Ex. No. | $R^1$ | n | Y | X | Z | Yield (% of theory) | $R_f$ (*) |
|---|---|---|---|---|---|---|---|
| 21 | H | 2 | H | $-CO_2H$ | H | quant. | 5.978 |
| 22 | 4-OH | 2 | H | $CH_3$ | H | 62 | 0.38 (A10)/6.690 |
| 23 | 6-OH | 2 | H | $CH_3$ | H | 76 | 0.26 (A5)/6.271 |
| 24 | 5-OH | 2 | H | $CH_3$ | H | 54 | 0.31 (A10)/6.414 |
| 25 | $6-OCH_2-C_6H_5$ | 2 | H | $CH_3$ | H | 76 | 0.21 (A5) |
| 26 | 6-OH | 2 | $-CH_3$ | H | H | 77 | 0.39 (A10)/6.252 |
| 27 | 6-OH | 2 | H | H | $CH_3$ | 81 | 0.44 (A10)/5.981 |
| 28 | OH | 2 | H | -Br | H | 48 | 6.64 |
| 29 | H | 2 | H | -Cl | H | 18 | 7.66 |
| 30 | 6-OH | 1 | H | $-CH_3$ | H | 84 | 0.07 (A1)/6.030 |
| 31 | H | 2 | $-CO_2H$ | $-OCH_3$ | H | 10 | 5.73 |
| 32 | H | 2 | H | Br | H | 70 | 0.51 (A5) |
| 33 | 6-OH | 2 | $-CO_2H$ | $-OCH_3$ | H | 64 | 5.001 |

## Claims

1. Benzofuranyl- and thiophenylmethylthio-alkanecarboxylic acid derivatives of the general formula

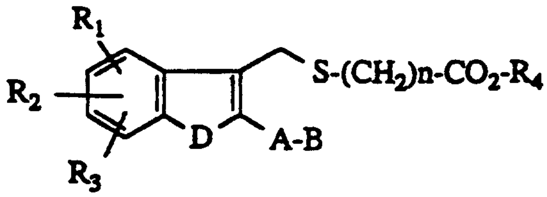

(I)

in which

| | |
|---|---|
| $R^1$, $R^2$ and $R^3$ | are identical or different and represent hydrogen, hydroxyl, straight-chain or branched alkoxy having up to 8 carbon atoms or benzyloxy, |
| n | represents a number 1, 2, 3, 4, 5 or 6, |
| $R^4$ | represents hydrogen, straight-chain or branched alkyl having up to 8 carbon atoms or phenyl, |
| A | represents a direct bond or the -CO- or $-CH_2-$ radical, |
| B | represents aryl having 6 to 10 carbon atoms or a 5-to 7-membered, saturated or unsaturated heterocycle having up to 3 heteroatoms from the series comprising N, S and O, which are optionally monosubstituted to trisubstituted by identical or different substituents from the series comprising carboxyl, halogen, cyano, phenyl, tetrazolyl, thiazolyl, and straight-chain or branched alkyl, alkoxy or alkoxycarbonyl each having up to 8 carbon atoms, or by a group of the formula $-CO-NR^5-SO_2-R^6$, in which |
| $R^5$ | denotes hydrogen or straight-chain or branched alkyl having up to 4 carbon atoms and |
| $R^6$ | denotes straight-chain or branched perfluoroalkyl having up to 6 carbon atoms, or denotes straight-chain or branched alkyl or alkenyl each having up to 6 carbon atoms, and each of which is optionally substituted by phenyl, where the phenyl radical can in turn be monosubstituted to trisubstituted by identical or different substituents from the series comprising halogen, carboxyl, cyano and nitro or by straight-chain or branched alkyl having up to 6 carbon atoms, denotes cycloalkyl having 3 to 7 carbon atoms, or denotes aryl having 6 to 10 carbon atoms or a 5-to 7-membered saturated or unsaturated heterocycle having up to 3 heteroatoms from the series comprising N, S and O, which are optionally monosubstituted to trisubstituted by identical or different substituents from the series comprising halogen, carboxyl, cyano and nitro or by straight-chain or branched alkyl having up to 6 carbon atoms, |
| D | represents an oxygen or sulphur atom |

and their salts.

**2.** Benzofuranyl- and thiophenylmethylthio-alkanecarboxylic acid derivatives according to Claim 1 in which

| | |
|---|---|
| $R^1$, $R^2$ and $R^3$ | are identical or different and represent hydrogen, hydroxyl, straight-chain or branched alkoxy having up to 6 carbon atoms or benzyloxy, |
| n | represents a number 1, 2, 3, 4 or 5, |
| $R^4$ | represents hydrogen, straight-chain or branched alkyl having up to 6 carbon atoms or phenyl, |
| A | represents a direct bond or the -CO- or $-CH_2-$ radical, |
| B | represents phenyl, pyridyl, thienyl or furyl, which are optionally monosubstituted to tri-substituted by identical or different substituents from the series comprising carboxyl, fluorine, chlorine, bromine, cyano, phenyl, tetrazolyl and thiazolyl, by straight-chain or branched alkyl, alkoxy or alkoxycarbonyl each having up to 6 carbon atoms or by a group of the formula $-CO-NR^5-SO_2R^6$, in which |
| $R^5$ | denotes hydrogen, methyl or ethyl and |
| $R^6$ | denotes straight-chain or branched alkyl or perfluoroalkyl each having up to 4 carbon atoms, or |

denotes cyclopropyl, cyclopentyl or cyclohexyl,
denotes benzyl, phenyl or pyridyl, which are optionally monosubstituted or disubstituted by identical or different substituents from the series comprising fluorine, chlorine, bromine, carboxyl, cyano and nitro or by straight-chain or branched alkyl having up to 4 carbon atoms,

D represents an oxygen or sulphur atom

and their salts.

**3.** Benzofuranyl- and thiophenylmethylthio-alkanecarboxylic acid derivatives according to Claim 1 in which

$R^1$, $R^2$ and $R^3$ are identical or different and represent hydrogen, hydroxyl, straight-chain or branched alkoxy having up to 4 carbon atoms or benzyloxy,

n represents a number 1, 2, 3 or 4,

$R^4$ represents hydrogen or straight-chain or branched alkyl having up to 4 carbon atoms,

A represents a direct bond or the -CO- or $-CH_2-$ radical,

B represents phenyl or pyridyl, which are optionally monosubstituted to trisubstituted by identical or different substituents from the series comprising carboxyl, cyano, chlorine, bromine, and straight-chain or branched alkyl, alkoxy or alkoxycarbonyl each having up to 4 carbon atoms or by a group of the formula $-CO-NR^5-SO_2R^6$, in which

$R^5$ denotes hydrogen or methyl and

$R^6$ denotes methyl, ethyl, propyl, trifluoromethyl, cyclopropyl or cyclohexyl, or denotes benzyl or phenyl, each of which is optionally monosubstituted or disubstituted by identical or different substituents from the series comprising fluorine, chlorine, bromine, carboxyl, methyl and ethyl,

D represents an oxygen or sulphur atom

and their salts.

**4.** Benzofuranyl- and thiophenylmethylthio-alkanecarboxylic acid derivatives according to Claim 1 for controlling diseases.

**5.** Process for the preparation of benzofuranyl- and thiophenylmethylthio-alkanecarboxylic acid derivatives of the general formula

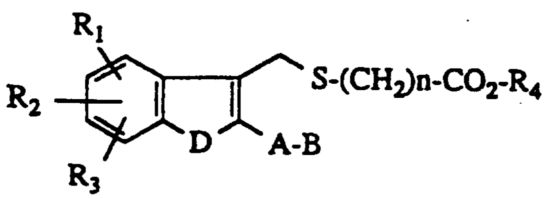
(I)

in which

$R^1$, $R^2$ and $R^3$ are identical or different and represent hydrogen, hydroxyl, straight-chain or branched alkoxy having up to 8 carbon atoms or benzyloxy,

n represents a number 1, 2, 3, 4, 5 or 6,

$R^4$ represents hydrogen, straight-chain or branched alkyl having up to 8 carbon atoms or phenyl,

A represents a direct bond or the -CO- or $-CH_2-$ radical,

B represents aryl having 6 to 10 carbon atoms or a 5-to 7-membered, saturated or unsaturated heterocycle having up to 3 heteroatoms from the series comprising N, S and O, which are optionally monosubstituted to trisubstituted by identical or different substituents from the series comprising carboxyl, halogen, cyano, phenyl, tetrazolyl, thiazolyl, and straight-chain or branched alkyl, alkoxy or alkoxycarbonyl each having up to 8 carbon atoms, or by a group of the formula $-CO-NR^5-SO_2-R^6$,

in which

$R^5$ denotes hydrogen or straight-chain or branched alkyl having up to 4 carbon atoms and

$R^6$ denotes straight-chain or branched perfluoroalkyl having up to 6 carbon atoms, or denotes straight-chain or branched alkyl or alkenyl each having up to 6 carbon atoms, and each of which is optionally substituted by phenyl, where the phenyl radical can in turn be substituted as described below for aryl,
denotes cycloalkyl having 3 to 7 carbon atoms, or
denotes aryl having 6 to 10 carbon atoms or a 5-to 7-membered saturated or unsaturated heterocycle having up to 3 heteroatoms from the series comprising N, S and O, which are optionally monosubstituted to trisubstituted by identical or different substituents from the series comprising halogen, carboxyl, cyano and nitro or by straight-chain or branched alkyl having up to 6 carbon atoms,

D represents an oxygen or sulphur atom

and their salts,
characterised in that compounds of the general formula

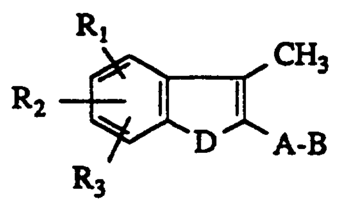

(II)

in which

$R^1$, $R^2$, $R^3$, A, B and D have the abovementioned meaning,

in the case in which $R^1$, $R^2$ and/or $R^3$ ≠ hydroxyl, are converted by reaction with N-bromosuccinimide and in the presence of a catalyst,
in the case in which $R^1$, $R^2$ and/or $R^3$ represents the hydroxyl function, these compounds are first blocked with typical hydroxyl protective groups and then converted with boron tribromide or hydrobromic acid in glacial acetic acid in inert solvents to the compounds of the general formula (III)

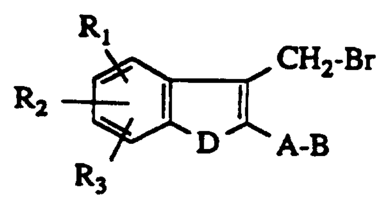

(III)

in which

$R^1$, $R^2$, $R^3$, A, B and D have the abovementioned meaning,

and in a next step the latter are reacted with compounds of the general formula (IV)

$HS-(CH_2)_n-CO_2R^7$ (IV)

in which

n has the abovementioned meaning

and

$R^7$ has the abovementioned meaning of $R^4$, but does not represent hydrogen,

in inert solvents, if appropriate in the presence of a base, under a protective gas atmosphere,
and in the case of the acids the esters are hydrolysed,
and in the case of the free hydroxyl functions ($R^1$, $R^2$ and/or $R^3$ = OH) the protective groups are removed by a customary method,
and in the case in which B represents one of the abovementioned cyclic radicals which are substituted by the group of the formula $-NR^5-SO_2R^6$, using sulphonamides of the formula (V)

$HNR^5-SO_2R^6$ (V)

in which

$R^5$ and $R^6$ have the abovementioned meaning,

starting from the free carboxylic acid, if appropriate in the presence of a base and/or of an auxiliary, an amidation follows.

**6.** Medicaments containing at least one benzofuranyl- or -thiophenylmethylthio-alkanecarboxylic acid derivative according to Claim 1.

**7.** Medicaments according to Claim 6 for controlling acute and chronic inflammatory processes.

**8.** Medicaments according to Claim 6 for reducing damage to infarct tissues after reoxygenation.

**9.** Process for the production of medicaments according to Claim 6, characterised in that the benzofuranyl- or thiophenylmethylthio-alkanecarboxylic acid derivatives are converted into a suitable administration form, if appropriate with customary auxiliaries and excipients.

**10.** Use of benzofuranyl- and thenylthio-alkanecarboxylic acid derivatives according to Claim 1 for the production of medicaments.

European Patent Office

# EUROPEAN SEARCH REPORT

Application Number

EP 92 12 2178

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,X | EP-A-0 146 243 (MERCK FROSST CANADA INC.)<br>* abstract; claims *<br>* in particular claim 1, page 142, definition of R1, item (4) *<br>--- | 1-4,6,9,10 | C07D307/80<br>C07D333/56<br>A61K31/34<br>A61K31/38 |
| A | EP-A-0 345 592 (TAKEDA CHEMICAL INDUSTRIES LTD.)<br>* abstract; claims *<br>* page 7, table 2, compounds 1, 2<br>----- | 1,6,9,10 | |
| | | | TECHNICAL FIELDS SEARCHED (Int. Cl.5) |
| | | | C07D |

The present search report has been drawn up for all claims

| Place of search | Date of completion of the search | Examiner |
|---|---|---|
| THE HAGUE | 02 APRIL 1993 | PAISDOR B. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document